(19) Europäisches Patentamt
European Patent Office
Office européen des brevets

(11) Veröffentlichungsnummer : **0 130 447**
**B1**

(12) # EUROPÄISCHE PATENTSCHRIFT

(45) Veröffentlichungstag der Patentschrift : **03.12.86**

(51) Int. Cl.⁴ : **C 07 D271/10**

(21) Anmeldenummer : **84106908.1**

(22) Anmeldetag : **16.06.84**

(54) **Verfahren zur Herstellung von 2-Aryl-4-acyl-1.3.4-oxdiazolonen-(5).**

(30) Priorität : **28.06.83 DE 3323283**

(43) Veröffentlichungstag der Anmeldung : **09.01.85 Patentblatt 85/02**

(45) Bekanntmachung des Hinweises auf die Patenterteilung : **03.12.86 Patentblatt 86/49**

(84) Benannte Vertragsstaaten : **DE FR GB IT**

(56) Entgegenhaltungen :
**US-A- 3 410 863**
**CHEMICAL ABSTRACTS, Vol. 98, No. 8, 21. Februar 1983, Columbus, Ohio, USA CHAU, NGUYEN; SAEGUSA, YASUO; IWAKURA, YOSHIO. "Synthesis of polyamide-quinazolinediones from 2,2'-disubstituted bis (1,3,4-oxadiazolin-5-ones) and aromatic bis-o-amino esters." Seite 7, Spalte 2, Abstract No. 54598t**
**CHEMICAL ABSTRACTS, Vol. 98, No. 18, 2. Mai 1983, Columbus, Ohio, USA SAEGUSA, YASUO; NAKAMURA, SHIGEO; NGUYEN CHAU; IWAKURA, YOSHIO. "Synthesis of polyamides from active 4,4'-diacylbis-2-aryl-1,3,4-oxadiazoline-5-thiones and -ones and diamines under mild conditions." Seite 6, Spalte 1, Abstract No. 143927s**
**CHEMICAL ABSTRACTS, Vol. 91, No. 19, 5. November 1979, Columbus, Ohio, USA HAYAMA, SHIGERU; TAKEISHI, MAKOTO: FUJII, SHOZO; WATANABE, YOSHIKI. "Solvolysis of azolides in the presence of poly (4(5)-vinylimidazole)." Seite 572, Spalte 1, Abstract No. 156918v**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Schmidt, Manfred, Dr.**
**Bodelschwinghstrasse 20**
**D-4150 Krefeld 1 (DE)**
Erfinder : **Meyer, Karl-Heinrich, Dr.**
**Deswatinesstrasse 89**
**D-4150 Krefeld 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 130 447 B1**

**Beschreibung**

2-Aryl-4-acyl-1.3.4-oxdiazolone-(5) ; insbesondere solche der Formel (I)

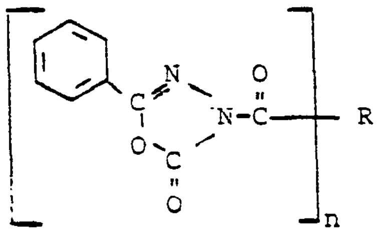

(I)

worin

R n-wertige $C_1$-$C_{18}$-Alkyl, $C_1$-$C_{18}$-Alkoxy, $C_6$-$C_{12}$-Cycloalkyl, $C_6$-$C_{12}$-Cycloalkoxy, $C_2$-$C_{18}$-Alkenyl, $C_6$-$C_{18}$-Cycloalkoxy, $C_6$-$C_{18}$-Aryl, $C_6$-$C_{12}$-Aryloxy, $C_7$-$C_{18}$-Arylalkyl, $C_7$-$C_{18}$-Arylalkyloxy, $C_7$-$C_{18}$-Alkylaryloxy, $C_7$-$C_{18}$-Alkylaryloxy-Reste

und

n 1 oder 2 bedeuten,

können als Treibmittel zur Herstellung von Strukturschäumen aus hochtemperaturbeständigen Kunststoffen verwendet werden. Beispielsweise lassen sich Schäume aus Polycarbonaten, Polyestercarbonaten, aromatischen Polyestern auf Basis von Bisphenolen und Iso-/Terephthalsäure, Polysulfonen, flüssigkristallinen aromatischen Polyestern mit anisotroper Schmelzphase, Polyethylenterephthalat, Polybutylenterephthalat, Polystyrol, ABS-Kunststoffen und aus Gemischen der vorgenannten Kunststoffe herstellen. Die Zersetzungstemperaturen der Oxdiazolone werden durch den Rest R bestimmt, so daß die Treibmittel entsprechend den Verarbeitungstemperaturen des zu verschäumenden Kunststoffs ausgewählt werden können.

Als Zerfallsprodukte entstehen Kohlendioxid und 1.3.4-Oxdiazole II gemäß :

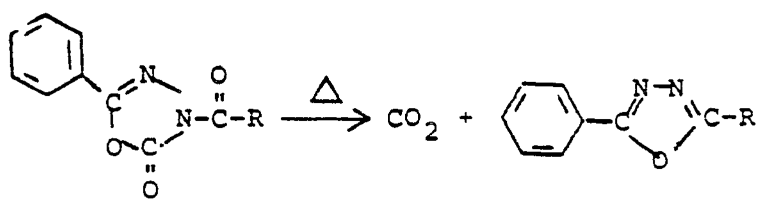

(I), n = 1 (II)

Die Herstellung von 2-Phenyl-1.3.4-oxdiazolon-(5) ist in Ber. *82*, S. 121-123 (1949) beschrieben. Hierzu wird ein Säurehydrazid mit Phosgen in wäßriger Lösung umgesetzt. Das Verfahren ist auch für 2-Alkylsubstituierte Oxdiazolone anwendbar. In der zitierten Literaturstelle wird auch die Umsetzung einiger Oxdiazolone in Pyridin mit Benzoylchlorid zu den entsprechenden 4-Acyl-Derivaten mitgeteilt.

Gegenstand der Erfindung ist ein verbessertes Verfahren zur Herstellung von 2-Aryl-4-acyl-1.3.4-oxdiazolonen-(5), das dadurch gekennzeichnet, ist, daß man ein aromatisches Carbonsäurehydrazid bei einem pH-Wert von 1,8-3 in einem Gemisch aus Wasser und Aceton bei Temperaturen von 0 °C bis 50 °C mit Phosgen umsetzt, dann den pH-Wert auf 9,5-11 erhöht und etwa 100 Mol-% eines Carbonsäurechlorids oder etwa 50 Mol-% eines Dicarbonsäurechlorids (bezogen auf 100 Mol-% Carbonsäurehydrazid) sowie gegebenenfalls $10^{-4}$ bis 1 Mol-% (bezogen auf 100 Mol-% Carbonsäurehydrazid) eines tertiären Amins oder Phosphins als Katalysator zufügt und die Umsetzung bei 0 °C bis 50 °C zu Ende führt.

Als aromatische Carbonsäurehydrazide sind beispielsweise geeignet : Benzoesäurehydrazid und Hydrazide von Naphthoesäuren, bevorzugt ist Benzoesäurehydrazid.

Als Carbonsäurechloride bzw. Dicarbonsäurechloride sind beispielsweise Verbindungen der Formeln III und IV einsetzbar.

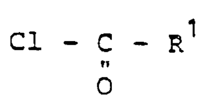

(III)

mit

$R^1$ = $C_1$-$C_{18}$ Alkyl, Alkoxy
= $C_6$-$C_{12}$ Cycloalkyl, Cycloalkyloxy
= $C_2$-$C_{18}$ Alkenyl, Alkenyloxy

= $C_6$-$C_{18}$ Aryl, Aryloxy
= $C_7$-$C_{18}$ Arylalkyl, Arylalkyloxy, Alkylaryl, Alkylaryloxy.

$$Cl-\underset{\underset{O}{\|}}{C}-R^2-\underset{\underset{O}{\|}}{C}-Cl \qquad (IV)$$

mit

$R^2$ = Einfachbindung, $C_1$-$C_{12}$ Alkylen, $C_6$-$C_{18}$ Arylen oder den Rest des Bisphenol A.

Besonders geeignete Carbonsäurechloride bzw. Dicarbonsäurechloride sind Benzoesäurechlorid, Naphthoesäurechloride, Isophthalsäuredichlorid, Terephthalsäuredichlorid, Bernsteinsäuredichlorid, Oxalsäuredichlorid, Malonsäuredichlorid.

Besonders geeignete Chlorkohlensäureester der Formel III bzw. Bis-chlorkohlensäureester der Formel IV sind Chlorkohlensäurephenylester, -ethylester, -propylester, der Bis-chlorkohlensäureester des Bisphenol A.

Das erfindungsgemäße Verfahren wird im folgenden anhand eines Beispiels näher erläutert.

Benzoesäurehydrazid wird in der 4-8 fachen Gewichtsmenge eines Wasser/Aceton-Gemisches suspendiert. Man leitet Phosgen in die bei 10 °C bis 25 °C gekühlte und gut gerührte Suspension (ca. 10 bis 20 Mol-% Überschuß) ein und hält den pH-Wert auf 1,8 bis 3,0 durch Zugabe von wäßriger Natriumhydroxidlösung. Die Phosgeneinleitung kann bei Normaldruck unter Verwendung eines Trockeneis/Methanol als Kühlmittel enthaltenden Kühlers oder nach der bevorzugten Verfahrensweise unter Verwendung einer geschlossenen Druck-Apparatur erfolgen. Nach beendeter Phosgeneinleitung werden $10^{-4}$ bis 1 Mol-% (bezogen auf Mole Benzoesäurehydrazid) eines tertiären Amins wie beispielsweise Triethylamin, N-Ethylpiperidin, Diazabicyclooctan oder eines tertiären Phosphins wie beispielsweise Triphenylphosphin oder ein $C_4$-$C_8$-Trialkylphosphin zugegeben und der pH-Wert mit wäßriger Natriumhydroxidlösung auf pH = 9,5 bis 11,0 eingestellt. Die verwendete NaOH-Menge muß mindestens der Menge eingesetzten Benzhydrazids äquimolar sein. Unter Kühlen (Reaktionstemperatur maximal 25 °C) wird eine Lösung von 100 Mol-% (bezogen auf Mole Benzoesäurehydrazid) eines Acylchlorids der allgemeinen Struktur (III) oder eine Lösung von 50 Mol-% eines Bisacyldichlorids der allgemeinen Struktur (IV) in Aceton (ca. 50 gew.-%ig) eingetragen und anschließend unter Rühren bei Raumtemperatur zu Ende umgesetzt.

Hierbei sinkt der pH-Wert auf 6,8-7,7. Das Reaktionsprodukt wird durch Filtration isoliert, mit Wasser chloridfrei gewaschen und im Vakuum bei 50-100 °C getrocknet.

Die resultierenden Ausbeuten sind ≥ 93 % der Theorie an 2-Aryl-4-acyl-1.3.4-oxdiazolon-(5)-derivaten der allgemeinen Struktur (I).

## Beispiel 1

Synthese von Di-[2-Phenyl-1.3.4-oxdiazolon-(5)-]-4-terephthalamid

50 g (0,368 Mol) Benzoesäurehydrazid (Fp. 112 °C) werden in 150 ml Aceton und 100 ml Wasser unter Rühren suspendiert. Unter Kühlung (Innentemperatur : 10-20 °C) werden in die Suspension 41,8 g (0,438 Mol) Phosgen (≙ 19 Mol-% Überschuß) eingeleitet, wobei durch gleichzeitige sukzessive Zugabe von 45 %iger Natronlauge (ca. 70 mol) der pH-Wert des Reaktionsgemisches auf 2 bis 2,5 gehalten wird. Eintragungsdauer : 20 Min. Man läßt anschließend unter $N_2$-Einleitung und intensivem Rühren 1/2 Std. nachreagieren. Durch Zugabe von 45 %iger Natronlauge (ca. 35-36 g) in die Suspension der farblosen Kristalle in wäßrigem Aceton wird der pH-Wert auf 10-10,5 eingestellt und die Innentemperatur auf 10-15 °C heruntergekühlt. Nach Zugabe von 0,014 g Triethylamin wird innerhalb von 20 Min. eine 50 %ige Lösung von 37,34 g (0,184 Mol) Terephthalsäuredichlorid in Aceton unter intensivem Rühren eingetragen, wobei durch Kühlung die Innentemperatur auf 10-15 °C gehalten wird. Hierbei sinkt der pH auf 6,8-7,1. Man läßt 2 Stdn. bei Raumtemperatur nachreagieren und isoliert das Reaktionsprodukt durch Filtration. Die farblosen Kristalle werden mit warmen Wasser chloridfrei gewaschen und im Vakuum bei 80 °C-100 °C getrocknet.

Ausbeute : 78 g (≙ 93 % der Theorie)
Fp. = 282 °C
$T_{zers.}$ = 285 °C
Unlöslich in Alkoholen, Dioxan, Ethylacetat, Chlorbenzol, Aceton.

## Beispiel 2

Synthese des 2-Phenyl-4-acetyl-1.3.4-oxdiazolon-(5)

50 g (0.368 Mol) Benzoesäurehydrazid werden in 50 ml Aceton sowie 150 ml Wasser unter Rühren suspendiert. Unter Kühlung (Innentemperatur : 10-20 °C) werden in die Suspension 41.8 g (0.438 Mol)

Phosgen (≙ 19 Mol-% Überschuß) eingeleitet, wobei durch gleichzeitige sukzessive Zugabe von 45 %iger Natronlauge (ca. 70 ml) der pH-Wert des Reaktionsgemisches auf 1,8 bis 2,0 gehalten wird. Man läßt anschließend 1/2 Std. bei Raumtemperatur nachreagieren und stellt dann durch Zugabe von 36 g 45 %iger Natronlauge den pH-Wert der Suspension auf 10,5 ein. Die Innentemperatur wird durch Kühlen auf 10 °C abgesenkt, und anschließend wird nach Zugabe von 0,02 g N-Ethylpiperidin eine Lösung von 28,89 g (= 0,368 Mol) Acetylchlorid in 30 ml Aceton innerhalb von 20 Min. zugegeben. Man läßt 1 Std. nachreagieren, wobei die Innentemperatur auf 20 °C und der pH-Wert auf 7,1 ansteigen. Das Reaktionsprodukt wird abfiltriert, mit Wasser chloridfrei gewaschen und im Vakuum bei 80 °C getrocknet.

Ausbeute : 71,32 g (≙ 95 % der Theorie)
Fp. 114 °C
Zersetzungspunkt : 200 °C.

Beispiel 3

Synthese von 2-Phenyl-4-phenoxycarbonyl-1.3.4-oxdiazolon-(5)

50 g (0,368 Mol) Benzoesäurehydrazid werden in 30 ml Aceton sowie in 180 ml Wasser unter Rühren suspendiert. Unter Kühlung (Innentemperatur : 10-20 °C) werden in die Suspension 41,0 g (0,430 Mol) Phosgen (≙ 18,6 Mol-% Überschuß) eingeleitet, wobei durch gleichzeitige dosierte Zugabe von 40 %iger Natronlauge (ca. 78 ml) der pH-Wert des Reaktionsgemisches auf 1,8 bis 2,0 gehalten wird. Man läßt anschließend 1/2 Std. bei Raumtemperatur nachreagieren und stellt dann durch Zugabe von 36 g 45 %iger Natronlauge den pH-Wert der Suspension auf 10,5 ein. Die Innentemperatur wird durch Kühlen auf 10 °C abgesenkt. Nach Zugabe von 0,05 g Triphenylphosphin wird eine Lösung von 57,59 g (0,368 Mol) Chlorameisensäurephenylester in 60 ml Aceton innerhalb von 30 Min. zugegeben. Man läßt 2 Stdn. nachreagieren, wobei die Innentemperatur auf 25 °C ansteigt und der pH-Wert auf 6,9 ansteigt. Das Reaktionsprodukt wird abfiltriert, mit Wasser chloridfrei gewaschen und im Vakuum bei 100 °C getrocknet.

Ausbeute : 101,7 g (≙ 96 % der Theorie)
Fp. = 147 °C
Zersetzungstemperatur = 286 °C.

**Patentansprüche**

1. Verfahren zur Herstellung von 2-Aryl-4-acyl-1.3.4-oxdiazolonen-(5), dadurch gekennzeichnet, daß man ein aromatisches Carbonsäurehydrazid bei einem pH-Wert von 1,8 bis 3,0 in einem Gemisch aus Wasser und Aceton als Lösungsmittel bei Temperaturen von 0 °C bis 50 °C mit Phosgen umsetzt, dann den pH-Wert auf 9,5 bis 11,0 erhöht und etwa 100 Mol-% eines Carbonsäurechlorids oder etwa 50 Mol-% eines Dicarbonsäurechlorids (bezogen auf 100 Mol-% Carbonsäurehydrazid) sowie gegebenenfalls $10^{-4}$ bis 1,0 Mol-% (bezogen auf 100 Mol-% Carbonsäurehydrazid) eines tertiären Amins oder Phosphins als Katalysator zufügt und die Umsetzung bei 0 °C bis 50 °C zu Ende führt.

2. Verfahren nach Anspruch 1, dadurch gekennzeichnet, daß man als aromatisches Carbonsäurehydrazid Benzoesäurehydrazid verwendet.

**Claims**

1. Process for the production of 2-aryl-4-acyl-1.3.4-oxdiazolones-(5), characterised in that an aromatic carboxylic acid hydrazide is reacted with phosgene at a pH value of 1.8 to 3.0 in a mixture of water and acetone as the solvent at temperatures of 0 °C to 50 °C, the pH value is then raised to 9.5 to 11.0 and about 100 mol % of a carboxylic acid chloride or about 50 mol % of a dicarboxylic acid chloride (based on 100 mol % of carboxylic acid hydrazide) and optionally $10^{-4}$ to 1.0 mol % (based on 100 mol % of carboxylic acid hydrazide) of a tertiary amine or phosphine, as a catalyst, are added and the reaction is completed at 0 °C to 50 °C.

2. Process according to Claim 1, characterised in that benzoic acid hydrazide is used as the aromatic carboxylic acid hydrazide.

**Revendications**

1. Procédé de préparation de 2-aryl-4-acyl-1.3.4-oxadiazolones-(5), caractérisé en ce que l'on fait réagir un hydrazide d'acide carboxylique aromatique, à un pH de 1,8 à 3,0, dans un mélange d'eau et d'acétone servant de solvant, à des températures de 0 à 50 °C, avec du phosgène, puis on porte le pH à un niveau de 9,5 à 11,0 et on ajoute environ 100 moles % d'un chlorure d'acide carboxylique ou environ 50 moles % d'un chlorure d'acide dicarboxylique (pour 100 moles % d'hydrazide d'acide carboxylique) et le

cas échéant $10^{-4}$ à 1,0 mole % (pour 100 moles % d'hydrazide d'acide carboxylique) d'une amine ou phosphine tertiaire servant de catalyseur et on termine la réaction à une température de 0 à 50 °C.

2. Procédé selon la revendication 1, caractérisé en ce que l'hydrazide d'acide carboxylique aromatique utilisé est l'hydrazide de l'acide benzoïque.